# EUROPÄISCHE PATENTSCHRIFT

(11) **EP 0 877 598 B1**
(45) Veröffentlichungstag und Bekanntmachung des Hinweises auf die Patenterteilung: **13.06.2001**
(21) Anmeldenummer: 97900555.0
(22) Anmeldetag: 03.01.1997
(51) Int. Cl.: A61K 9/00, A01N 25/00, A23L 1/0522

(54) **ANTHELMINTHISCHE PASTE**
ANTHELMINTIC PASTE
PATE ANTHELMINTHIQUE

(30) Priorität: 16.01.1996 DE 19601263
(43) Veröffentlichungstag der Anmeldung: 18.11.1998
(73) Patentinhaber: BAYER AG, 51368 Leverkusen (DE)
(72) Erfinder: KALBE, Jochen, D-42799 Leichlingen (DE); SCHNABEL, Helmut, D-51519 Odenthal (DE)
(86) Internationale Anmeldenummer: EP9700006
(87) Internationale Veröffentlichungsnummer: WO9725976

(56) Entgegenhaltungen:
- EP-A- 0 279 343
- CH-A- 685 370
- US-A- 4 117 156
- US-A- 4 447 414

## Beschreibung

Die vorliegende Erfindung betrifft anthelmintische Pasten für Pferde, Hunde und Katzen auf Basis von Hexahydropyrazinoderivaten.

Praziquantel-haltige Pasten sind bekannt aus EP-A 279 343. Nachteilig bei diesen Pasten ist jedoch ihr hoher Wassergehalt. Die Pasten müssen daher vor Befall durch pathogene Bakterien durch Konservierungsmittel geschützt werden. Auch das homogene Einmischen der schwer wasserlöslichen Wirkstoffe bereitet oft Schwierigkeiten.

Die vorliegende Erfindung betrifft anthelmintische Pasten für Pferde, Hunde und Katzen auf der Basis von Hexahydropyrazinoderivaten, die dadurch gekennzeichnet sind, daß sie neben dem Wirkstoff als Hilfsstoffe zur Pastenbildung Maisstärke und Glycerin enthalten.

Es handelt sich um eine Formulierung die
1. nur aus wenigen Komponenten aufgebaut ist,
2. aus preiswerten Komponenten aufgebaut ist,
3. ohne besondere Konservierungsmittel auskommt,
4. einfach herzustellen ist.

Bevorzugt sind erfindungsgemäße Mischungen, die als Hexahydropyrazinderivate Praziquantel und Epsiprantel enthalten. Besonders bevorzugt ist Praziquantel.

Der Wirkstoff ist in den erfindungsgemäßen Formulierungen in Gewichtskonzentrationen von 5 bis 30 %, bevorzugt von 10 bis 20 % enthalten.

Maisstärke ist in den erfindungsgemäßen Formulierungen in Gewichtskonzentrationen von 10 bis 30 %, bevorzugt von 15 bis 25 % enthalten.

Glycerin ist in den erfindungsgemäßen Formulierungen in Gewichtskonzentrationen von 40 bis 80 %, bevorzugt von 55 bis 75 % enthalten. Die Einzelkomponenten ergänzen sich zu 100 %. Das Verhältnis Maisstärke zu Glycerin wird so gewählt, daß eine leicht verarbeitbare, verpackbare und anwendungsfähige Formulierung entsteht.

Die erfindungsgemäßen Formulierungen können neben den Hexahydropyrazinonen noch weitere Wirkstoffe enthalten. Dazu zählen Phenylguanidine, Benzimidazole oder Tetrahydropyrimidine.

### Als Phenylguanidine seien solche der Formel (I) genannt:

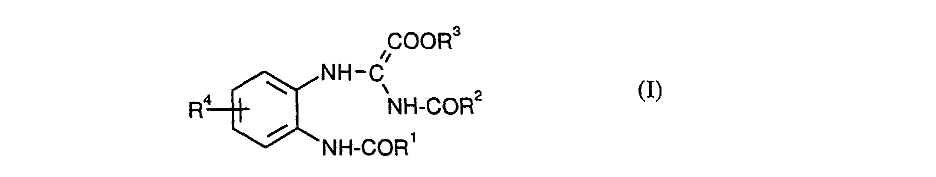

in welcher
- R¹: für Wasserstoff, gegebenenfalls substituiertes Alkyl, Cycloalkyl, Alkoxy, Aryl oder Amino steht,
- R²: für Wasserstoff, gegebenenfalls substituiertes Alkyl, Cycloalkyl, Alkenyl, Alkinyl, Aryl, Alkoxy, Alkenoxy, steht,
- R³: für Wasserstoff, gegebenenfalls substituiertes Alkyl, Cycloalkyl, Alkenyl, Alkinyl, Aryl, steht,
- R⁴: für Alkyl, Alkoxy, Phenoxy, Alkylthio, Phenylthio, Phenylsulfinyl, Phenylsulfonyl, Benzoyl steht, die gegebenenfalls substituiert sind durch Halogen, Alkoxy, Alkylthio, Halogenalkoxy, Halogenalkylthio.

Als Benzimidazole seien solche der Formel (II) genannt: 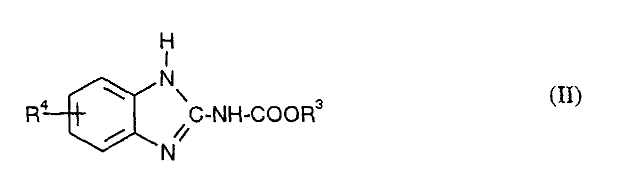 in welcher R³ und R⁴ die bei den Verbindungen der Formel (I) angegebene Bedeutung haben:

Als Tetrahydropyrimidine seien solche der Formel (III) genannt: 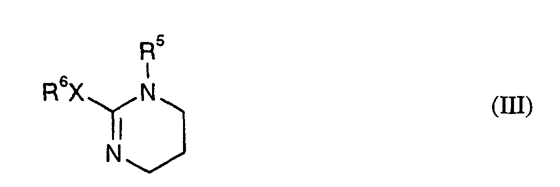 in welcher
- R⁵: für Wasserstoff oder Alkyl steht,
- R⁶: für gegebenenfalls substituiertes Phenyl oder Thienyl steht,
- X: für -(CH₂)₂₋₃-, -CH=CH- steht.

Zu den Phenylguanidinen gehören z.B. Febantel, Netobimin.

Zu den Benzimidazolen gehören z.B. Febendazol, Albendazol, Oxibendazol, Oxfendazol, Mebendazol, Flubendazol, Parbendazol, Luxabendazol.

Zu den Tetrahydropyrimidinen gehören z.B. Pyrantel, Morantel, Oxantel.

Die erfindungsgemäßen Pasten eignen sich bei günstiger Warmblütertoxizität zur Bekämpfung von pathogenen Endoparasiten die bei Pferden in der Tierhaltung und Tierzucht vorkommen. Sie sind dabei gegen alle oder einzelne Entwicklungsstadien der Schädlinge sowie gegen resistente und normal sensible Arten wirksam. Zu den pathogenen Endoparasiten zählen Cestoden, Trematoden, Nematoden, Acantocephalen, insbesondere:
Aus der Ordnung der Pseudophyllidea z.B.: Diphyllobothrium spp., Spirometra spp., Schistocephalus spp..

Aus der Ordnung der Cyclophyllidea z.B.: Mesocestoides spp., Anoplocephala spp., Paranoplocephala spp., Moniezia spp., Taenia spp., Echinococcus spp., Hydatigera spp., Diorchis spp., Dipylidium spp., Joyeuxiella spp., Spyrometra spp..

Aus der Unterklasse der Digenea z.B.: Schistosoma spp., Fasciola spp., Dicrocoelium spp., Opisthorchis spp..

Aus der Ordnung der Enoplida z.B.: Trichuris spp., Capillaria spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Poteriostomum spp., Cyclicocyclus spp., Stephanurus spp., Ancyclostoma spp., Uncinaria spp., Cyathostomum spp., Metastrongylus spp., Dictyocaulus spp., Muellerius spp., Protostrongylus spp., Elaphostrongylus spp., Parelaphostrongylus spp., Crenosoma spp., Paracrenosoma spp., Filaroides spp., Parafilaroides spp., Marshallagia spp., Hyostrongylus spp., Ollulanus spp., Craterostomum spp., Cyclicodontophorus spp., Hyalocephalus spp., Cylindropharynx spp., Caballonema spp., Elaeophorus spp., Dirofilaria spp., Onchocerca spp., Setaria spp..

Aus der Ordnung der Oxyurida z.B.: Oxyuris spp., Enterobius spp..

Aus der Ordnung der Ascaridia z.B. Ascaris spp., Toxascaris spp., Toxocara spp., Parascaris spp., Probstmangria spp..

Aus der Ordnung der Spirurida z.B.: Thelazia spp., Habronema spp., Draschia spp., Dracunculus spp..

Die Anwendung kann sowohl prophylaktisch als auch therapeutisch erfolgen.

Die bevorzugte Anwendungskonzentration der erfindungsgemäßen Mischung liegt pro kg Lebendgewicht bei 1 bis 300 mg, bevorzugt bei 5 bis 50 mg Hexahydropyrazin.

Weitere anthelminthische Wirkstoffe können zur Anwendung in einer Aufwandmenge pro kg von 0,1 bis 20 mg, bevorzugt 1 bis 10 mg, besonders bevorzugt ca. 5 mg, zugesetzt werden.

### Beispiel 1

Paste zur oralen Applikation oder zum Mischen unter das Futter

### Zusammensetzung:

| | |
|---|---|
| Praziquantel | 20,0 g |
| Maisstärke | 15,0 g |
| Glycerin | 65,0 g |

### Herstellung:

Die Komponenten werden zusammengerührt. Es entsteht eine Paste die in entsprechende Applikatoren gefüllt werden kann.

## Patentansprüche

1. Anthelminthische Pasten für Pferde, Hunde und Katzen auf der Basis von Hexahydropyrazinoderivaten, die dadurch gekennzeichnet sind, daß sie neben dem Wirkstoff als Hilfsstoffe zur Pastenbildung Maisstärke und Glycerin enthalten.

2. Anthelminthische Pasten für Pferde, Hunde und Katzen gemäß Anspruch 1, dadurch gekennzeichnet, daß der Wirkstoff in Gewichtskonzentrationen von 5 bis 30 % enthalten ist, Maisstärke in Gewichtskonzentrationen von 10 bis 30 % enthalten ist, Glycerin in Gewichtskonzentrationen von 40 bis 80 % enthalten ist.

## Claims

1. Hexahydropyrazino-derivative-based anthelmintic pastes for horses, dogs and cats, which are characterized in that they comprise corn starch and glycerol as adjuvants for paste formation, in addition to the active compound.

2. Anthelmintic pastes for horses, dogs and cats according to Claim 1, characterized in that they comprise the active compound in weight concentrations of 5 to 30%, corn starch in weight concentrations of 10 to 30% and glycerol in weight concentrations of 40 to 80%.

## Revendications

1. Pâtes anthelminthiques pour chevaux, chiens et chats, à base de dérivés d'hexahydropyrazine, qui sont caractérisées en ce qu'elles contiennent, outre l'agent actif, de l'amidon de maïs et de la glycérine comme auxiliaires pour la formation de la pâte.

2. Pâtes anthelminthiques pour chevaux, chiens et chats suivant la revendication 1, caractérisées en ce que l'agent actif est présent en une concentration pondérale allant de 5 à 30%, l'amidon de maïs en une concentration pondérale allant de 10 à 30% et la glycérine en une concentration pondérale allant de 40 à 80%.
